# EUROPEAN PATENT APPLICATION

(11) **EP 4 261 832 A1**
(43) Date of publication of application: **18.10.2023**
(21) Application number: 22187417.5
(22) Date of filing: 28.07.2022
(51) Int. Cl.: G16H 10/60, G16H 30/20, G16H 50/30

(54) **PATIENT MONITORING DEVICE**

(30) Priority: 15.04.2022 US 202263331295 P
(71) Applicant: Koninklijke Philips N.V., 5656 AG Eindhoven (NL)
(72) Inventor: SUTTON, Jonathan Thomas, Eindhoven (NL); BERA, Deep, Eindhoven (NL); BHARAT, Shyam, 5656 AG Eindhoven (NL); BULUT, Murtaza, 5656 AG Eindhoven (NL); HUIJBREGTS, Laurentia Johanna, Eindhoven (NL)
(74) Representative: Philips Intellectual Property & Standards

(57) **Abstract**

According to an aspect, there is provided a computer implemented method performed by a patient monitoring device. The method comprises using a patient monitoring model to determine a health parameter for a subject. The method further comprises using one or more outputs of the patient monitoring model to determine a search query with which to query a database of medical images in order to retrieve a matching medical image relevant to the determined health parameter.

## Description

### FIELD OF THE INVENTION

The disclosure herein relates to a patient monitoring device, and a computer implemented method performed by a patient monitoring device.

### BACKGROUND OF THE INVENTION

The disclosure herein relates to monitoring of a patient in a clinical setting such as a hospital, clinic, care home or any other medical facility. In such facilities patients are often monitored in an automated manner. For example, vital signs, such as heart rate, blood pressure, and SpO2 rate, amongst others, may be monitored using sensors attached to the patient.

Furthermore, clinical decision support tools are increasingly common in hospital settings. Such tools (or models) exchange data with a database such as an electronic medical records (EMR) or patient monitoring database during the patient's stay and generate predictions from the monitored vital signs. Predictions may e.g. relate to a deterioration event and may be used to trigger alarms on a patient monitor. Patient monitors, such as bedside monitors, tele-health monitors (for monitoring a patient remotely) and central stations (where data from multiple patient monitoring systems is displayed) are often the main vital sign visualization dashboards for a critical care team.

### SUMMARY OF THE INVENTION

In clinical settings, care is often centered around workflows. For example, there are ultrasound workflows associated with obtaining, processing and storing ultrasound images obtained in an ultrasound examination. Ultrasound workflows are well defined in cardiology and radiology and often revolve around modality worklists and Digital Imaging and Communications in Medicine DICOM studies which comprise a plurality of images.

There are also separate workflows associated with patient monitoring in a critical care environment, for example, pertaining to obtaining storing and processing vital sign measurements.

The workflows are put in place to standardize care and ensure a continuous quality of care is met across different patients. Workflows also ensure rigorous record keeping.

A downside of workflows is that they can be difficult to combine, which can result in information not being optimally stored or retrieved. It is an object of the disclosure herein to improve on the aforementioned problems.

Thus according to a first aspect, there is provided a computer implemented method performed by a patient monitoring device. The method comprises: using a patient monitoring model to determine a health parameter for a subject; and using one or more outputs of the patient monitoring model to determine a search query with which to query a database of medical images in order to retrieve a matching medical image relevant to the determined health parameter.

According to a second aspect there is an apparatus comprising: a memory comprising instruction data representing a set of instructions; and a processor configured to communicate with the memory and to execute the set of instructions, wherein the set of instructions, when executed by the processor, cause the processor to: use a patient monitoring model to determine a health parameter for a subject; and use one or more outputs of the patient monitoring model to determine a search query with which to query a database of medical images in order to retrieve a matching medical image relevant to the determined health parameter.

According to a third aspect there is a patient monitoring device comprising the apparatus of the second aspect, and further comprising: a display. The processor is further caused to: obtain the matching medical image from the database of medical images; and send an instruction to the display to cause the display to make the matching medical image available for rendering on the display.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the first aspect.

Thus, there is provided, methods and apparatus to select medical image sequences for display on a patient monitoring display in an automated manner. As noted above, the disclosure herein is made in response to the challenge in combining the workflows of ultrasound and patient monitoring in a critical care environment. The disclosure herein provides a novel approach to the challenge of a Patient Monitor being able to "Pull" relevant Ultrasound Data from a server. The advantage of this approach is that it enables relevant images to be obtained/"pulled" by a patient monitor in response to a prediction or calculation made by a clinical decision support tool so as to provide images relevant to the prediction/calculation made by the clinical decision support tool and to aid a clinician in interpreting the result.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments herein;
Fig. 2 shows a patient monitoring device according to some embodiments herein;
Fig. 3 shows a method according to some embodiments herein;
Fig. 4 shows an example system according to some embodiments herein; and
Fig. 5 shows an example signal flow between the components illustrated in Fig. 4.

### DETAILED DESCRIPTION OF EMBODIMENTS

As described above, the disclosure herein was inspired by a cross value stream initiative in ultrasound and patient monitoring. It was recognized by the inventors herein that there exists a challenge in combining the workflows of ultrasound and patient monitoring in a critical care environment. Ultrasound workflows are well defined in e.g. cardiology and radiology and comprise of modality worklists and DICOM studies which comprise a plurality of images. Measurements produce separate entities called structured reports, similarly to Computed tomography (CT)/Magnetic Resonance (MR). The data are stored in institutional DICOM storage entities or picture archiving and communication system (PACS) archives. In a critical care environments, these workflows are adopted slowly as more care providers become proficient in acquisition, and as new technology helps to improve the reproducibility and continual nature of the echo-derived vital signs. Dedicated storage of image data in critical care currently tends to occur episodically and is often not stored permanently, but when it is stored, it is often in separate DICOM storage entities on premise.

Patient monitoring information on the other hand is stored in various locations including at the bedside as well as at central information centers. Patient monitoring information tends to be stored using industry standard formats such as the Health Level Seven (HL7) format which is a set of international standards for transferring clinical and administrative data between software applications used by healthcare providers.

Patient monitors such as bedside monitors, telehealth monitors and central stations tend to be the de facto vital sign visualization dashboards for a care team (such as a critical care team) in a hospital. As described in more detail below, the disclosure herein relates to improved patient monitors, tele-health monitors and/or central stations that are capable of displaying medical image(s) that are relevant to the current status of the patient(s) being monitored. The disclosure herein thus enables "Monitoring Pull": e.g. the ability of a patient monitor to filter and display relevant image data for monitoring insights.

In brief, the invention herein enables a patient monitoring system to filter and retrieve ultrasound images and measurements that are clinically relevant to the output of a clinical decision support tool. In short, a clinical decision support tool, as described above, exchanges data with the Patient Information Centre during the patient's stay and generates a prediction, such as a prediction of a deterioration event. The outputs of the clinical decision support tool are used to determine a list of DICOM query parameters that can be used to obtain images relevant to the predicted deterioration event. In some embodiments, the list of parameters could be generated as part of the tool's output, or performed separately by a predefined lookup table, or a user configurable lookup table.

Thus, there is provided a mechanism for "pulling" relevant images associated with the output of a clinical decision making tool to a patient monitor.

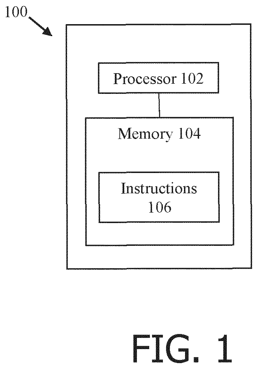

Turning now to Fig. 1 in some embodiments there is an apparatus 100, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 100 may be for use in monitoring a patient, e.g. in a clinical setting such as a hospital. More specifically, the set of instructions, when executed by the processor, cause the processor to: use a patient monitoring model to determine a health parameter for a subject; and use one or more outputs of the patient monitoring model to determine a search query with which to query a database of medical images in order to retrieve a matching medical image relevant to the determined health parameter.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the determined health parameter, the search query, the matching medical image and/or any other information or data received, calculated or determined by the processor 102 of the apparatus 100 or from any interfaces, memories or devices that are external to the apparatus 100. The processor 102 may be configured to control the memory 104 to store the determined health parameter, the search query, the matching medical image and/or the any other information or data.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may comprise a battery or other power supply for powering the apparatus 100 or means for connecting the apparatus 100 to a mains power supply. The apparatus 100 may further comprise a communications interface, e.g. for communicating wirelessly with other computing apparatuses.

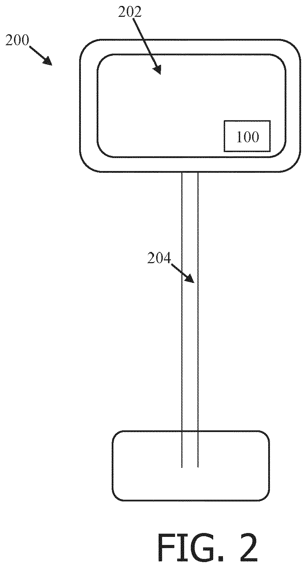

The apparatus 100 may be comprised in a patient monitor, such as the patient monitor 200 illustrated in Fig 2. The patient monitor 200 comprises a display 202. In this example, the processor 102 is further caused to obtain the matching medical image from the database of medical images, and send an instruction to a display to cause the display to make the matching medical image available for rendering on the display the matching medical image.

A display 202 may comprise, for example, a computer screen, such as a liquid crystal display (LCD), or any other type of display. A patient monitor may further comprise other components, for example, a stand 204, and/or a user input device, such as a keyboard, mouse, one or more buttons, or any other input device that enables a user to interact with the apparatus, for example, to cause the display to display the matched image.

Furthermore, the patient monitoring device 200 may comprise one or more sensors, e.g. for obtaining vital signs or other information about a patient being monitored. Examples of sensors include but are not limited to, an SpO2 monitor, a heart-rate monitor and a blood pressure monitor.

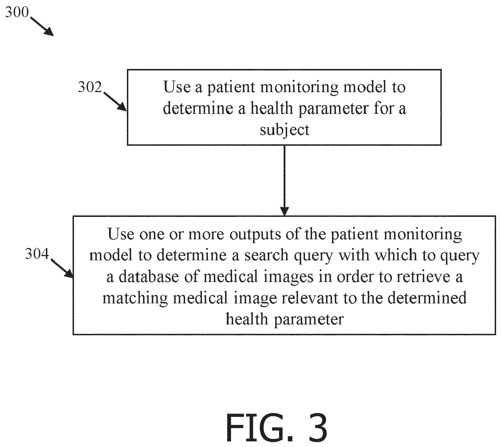

Turning to Fig. 3, there is a computer implemented method 300 performed by a patient monitoring device. Embodiments of the method 300 may be performed, for example by an apparatus such as the apparatus 100 described above.

Briefly, in a first step 302, the method 300 comprises: using a patient monitoring model to determine a health parameter for a subject. In a second step 304 the method 300 comprises using one or more outputs of the patient monitoring model to determine a search query with which to query a database of medical images in order to retrieve a matching medical image relevant to the determined health parameter.

As used herein a patient monitoring device (or patient monitor) may be, for example, a bedside monitor, or a central station e.g. a central computer unit for receiving and displaying information for monitoring a plurality of patients. In other embodiments, the patient monitoring device may be a remote-monitoring device such as a tele-health monitor. An example of a tele-health monitor is a tele-Intensive care unit (ICU) monitor.

As noted above, in step 302, a patient monitoring model is used to determine a health parameter for a subject.

A patient monitoring model as used herein may be a clinical decision tool, clinical decision support algorithm, or any other model or process that can be used to process monitored information from a patient.

A patient monitoring model may, for example, take one or more vital signs as input and output a health parameter. The health parameter may be determined, e.g. calculated or predicted by the patient monitoring model. A patient monitoring model may determine the health parameter in any manner, for example, using a look-up table, a sequence of thresholds or if/then statements, equations, using machine learning, or in any other manner.

In some embodiments, the health parameter is a score. As such, the score may be derived from patient data available to the patient monitoring device. For example, a score may be any metric used to determine whether a patient's condition is, improving, deteriorating or stable. A score may be linked to a specific structure, parameter, time of measurement, or parameter value. Examples of scores include, but are not limited to: a Hemodynamic Stability Index score; a Hypotensive Predictive Index score; an acute heart failure prediction index; a score linked to a patient monitoring alarm or patient monitoring trend.

Examples of patient monitoring modes include but are not limited to: models for determining a Hemodynamic Stability Index score, a Hypotensive Predictive Index score, or an acute heart failure prediction index.

One primary need for ultrasound in critical care is to help manage longitudinal management of the patient's cardiovascular physiology for improved hemodynamics. An example of this is the patient-specific Frank-Starling relation of the heart, which describes the relationship between cardiac preload and output. Ultrasound is a trusted non-invasive tool to measure this in real time on a beat-to-beat basis.

The skilled person will appreciate however that these are merely examples and that the method 300 applies equally to any patient monitoring model.

In step 304, the method comprises using one or more outputs of the patient monitoring model to determine a search query with which to query a database of medical images in order to retrieve a matching medical image relevant to the determined health parameter.

Although some of the examples herein describe Ultrasound (US) images, the medical images in the database of medical images may be any imagine modality. Example medical images include, but are not limited to, a computed tomography (CT) image (for example, from a CT scan) such as a C-arm CT image, a spectral CT image or a phase contrast CT Image, an x-ray image (for example, from an x-ray scan), a magnetic resonance (MR) image (for example, from an MR scan), an ultrasound (US) image (for example, from an ultrasound scan), fluoroscopy images, nuclear medicine images, or any other medical image.

The medical images may be stored in a format containing meta information (e.g. meta data). For example, in the form of an image header. The skilled person will appreciate that this is merely an example however, and that metadata can be stored in other ways, for example, using strings, images, event hyperlinks, coding objects, and/or macros linked to applications.

In some embodiments, the medical images may be in the Digital Imaging and Communications in Medicine (DICOM) format. The DICOM format is described in the paper by Dandu Ravi Varma (2012) entitled, "Managing DICOM images: Tips and tricks for the radiologist"; Indian J Radiol Imaging. 2012 Jan-Mar; 22(1): 4-13. DICOM metadata can be read and/or searched using various tools and coding languages. As an example, in MathWorks, metadata from DICOM file "Filename.dcm" can be read using the following command: metadata = dicominfo('Filename.dcm'). It will be appreciated that this is merely an example however and that the medical images may be stored in other formats, for example, the Flexible Image Transport System (FITS) format, or any other format comprising searchable meta information.

In some embodiments, the medical images may be stored in an institutional DICOM storage entity or picture archiving and communication system (PACS) archive. Thus, the database of medical images may be a DICOM database, a PACS database, or any other database containing medical images. The database of medical images may be referred to as an electronic medical records (EMR) database or a patient monitor database. The database of medical images may be stored or hosted locally, e.g. forming part of a hospital network. Alternatively, the patient database of medical images may be made available via the cloud.

The medical images may be associated with accompanying structured reports comprising measurements and/or annotations (such as calculations) of a feature in the associated medical image. Structured reports may be generated in a standardized manner, e.g. as part of an ultrasound workflow.

In step 302, one or more outputs of the patient monitoring model are used to determine a search query with which to query the database of medical images in order to retrieve a matching medical image relevant to the determined health parameter. In this sense a matching medical image is one that satisfies the search query.

In some examples, at least one of the one or more outputs of the patient monitoring model is formatted in a manner for use directly as a Digital Imaging and Communications in Medicine, DICOM, search query. For example, the patient monitoring model may be configured to output a search query.

Using the mitral valve as an example, the query can contain the words "mitral valve", or additionally (or alternately) can specify a particular image view (e.g., an "apical 4 chamber (A4C) view") that indirectly ensures visualization of the mitral valve. Additional options can be to filter by Ultrasound (US) acquisition parameters such as depth, focal zone, mode (Bmode/color/PW) etc. For example, an A4C view acquired at a 18 cm depth (vs, say, an 8 cm depth) is more likely to fully include visualization of the mitral valve.

DICOM query format can be found in the online documentation: "DICOM PS3.3 2022b - Information Object Definitions" which is a DICOM^{®} publication by the DICOM Standards Committee (2022). For example, the section: DICOM PS3.17 2022b - Explanatory Information: "*N.3.5 Left Ventricle Output"* relates to the DICOM standard for left ventricle performance.

The following code demonstrates a python example of querying for a DICOM study using a DICOM tag (PatientName) using the DICOM web standard. In this example, the "params" may be output by the patient monitoring model for use in the query.

```
==============================================================================
 def dicomweb_search_studies(project_id, location, dataset_id, dicom_store_id):
 ʺʺʺHandles the GET requests specified in the DICOMweb standard.
 See https://github.com/GoogleCloudPlatform/python-docs-samples/tree/main/healthcare/api
 -client/v1/dicom
 before running the sample."""
 # Imports Python's built-in "os" module
 import os
 
 # Imports the google.auth.transport.requests transport
 from google.auth.transport import requests
 
 # Imports a module to allow authentication using a service account
 from google.oauth2 import service_account
 
 # Gets credentials from the environment.
 credentials = service_account.Credentials.from_service_account_file(
  os.environ["GOOGLE_APPLICATION_CREDENTIALS"]
 )
 scoped _credentials = credentials.with_scopes(
   [https://www.googleapis.com/auth/cloud-platform]
 )
 # Creates a requests Session object with the credentials.
 session = requests.AuthorizedSession(scoped_credentials)
 
 # URL to the Cloud Healthcare API endpoint and version
 base_url = https://healthcare.googleapis.com/vl
 
 # TODO(developer): Uncomment these lines and replace with your values.
 # project_id = 'my-project' # replace with your GCP project ID
 # location = 'us-central1' # replace with the parent dataset's location
 # dataset_id = 'my-dataset' # replace with the parent dataset's ID
 # dicom_store_id = 'my-dicom-store' # replace with the DICOM store ID
 url = "{}/projects/{}/locations/{}".format(base_url, project_id, location)
 
 dicomweb_path = " {}/datasets/{ }/dicomStores/{ }/dicomWeb/studies" .format(
  url, dataset _id, dicom_store_id
 )
 
 # Refine your search by appending DICOM tags to the
 # request in the form of query parameters. This sample
 # searches for studies containing a patient's name.
 params = {"PatientName": "Sally Zhang"}
 
 response = session.get(dicomweb_path, params=params)
 
 response .raise_for_status()
 
 print("Studies found: response is {}".format(response))
 
 # Uncomment the following lines to process the response as JSON.
 # patients = responsej.son()
 # print('Patients found matching query:')
 # print(json.dumps(patients, indent=2))
 
 # return patients
=================================================================
```

In some embodiments, at least one of the one or more outputs of the patient monitoring model is formatted as a string or image and the string or image is used in the search query.

In other examples, the one or more outputs of the patient monitoring model may be converted into the search query using a look-up table, a machine learning (ML) model, a mapping procedure, a Mathematical function, or any other method of converting an output into a DICOM search Query. It will be appreciated that look-up tables and mapping functions are pre-configurable by a human engineer who may specify an appropriate DICOM query for different circumstances. Similarly ML models (such as neural networks) and the like may be trained using training data comprising training examples where each training example comprises i) an example output of the patient monitoring model and ii) a ground truth DICOM query that should be made for said example output.

In this way, the outputs of a patient monitoring model may be formatted into a query for a database in order to search and retrieve medical images relevant to the health parameter.

Following step 304, the method 300 may further comprise obtaining the matching medical image from the database of medical images, and sending an instruction to a display to make the matching medical image available for rendering on the display. The matching medical image may be automatically rendered, or rendered upon request by a user of the patient monitor.

In some embodiments, step 304 may be performed responsive to certain criteria being met, e.g. responsive to a trigger.

For example, step 304 may be performed responsive to the determined health parameter triggering an alert on the patient monitoring device. In such an example, when an alert is triggered due to the health parameter (or value thereof), the patient monitor may automatically query the database of medical images in order to obtain medical images that are relevant to the alert. These may thus be displayed on the patient monitor in order to aid a clinician in determining whether action is required.

In another example, step 304 might be triggered at another threshold of the health parameter than the threshold to trigger an alert on the patient monitor. For example a first threshold may be used to trigger performance of step 304 and a second threshold may be used to trigger an alarm.

In some embodiments, step 304 may be performed responsive to the determined health parameter being indicative of a deterioration of the patient. E.g. the health parameter (or value thereof) may be compared to a criteria and if the criteria indicates (likely) deterioration of the patient, then this may trigger performance of step 304. Thus, if a patient begins to deteriorate, the patient monitor may pull medical images relevant to the particular deterioration condition, thus providing all relevant information to the clinician on the patient monitor.

In this way, the information produced by the image processing workflow (e.g US workflow) may be joined up with a patient monitoring workflow in order to provide a clinician with the most relevant information on a patient and to help them make the most informed decisions about the patient's care.

In another embodiment, more than one query may be formatted. For example, the patient monitoring model may query the database of medical images as part of step 202 (e.g. as part of the step of using a patient monitoring model to determine a health parameter for a subject). For example, the method 300 may comprise generating first outputs to generate a first search query, the results of which are used by the patient monitoring model to produce the health parameter (which in this embodiment may be thought of as a second, updated or final output) that may be displayed to user. As a result of the second output, a second query for search can be also generated according to step 304. In this way, an initial output of a patient monitoring model may be used to query a database of medical images in order to obtain information for use in determining the final health parameter.

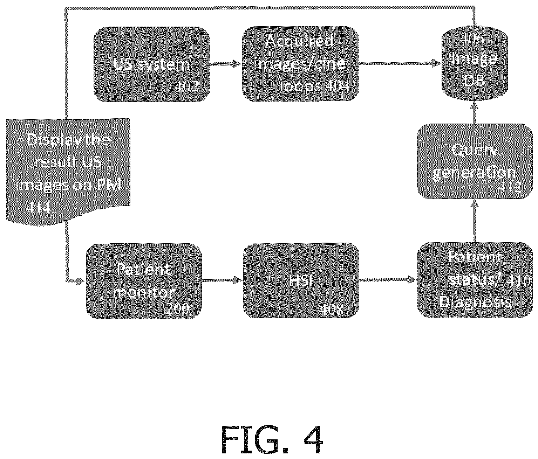

Turning now to Fig. 4 which shows how a patient monitor 200 may interface with other elements in a hospital system in order to perform the method 300 described above according to some embodiments herein. In this embodiment, the medical images are US images, stored in the DICOM image format. It will be appreciated that these are merely examples however and that the example in Fig. 4 applies equally to other image modalities and other patient monitoring models.
The embodiment in Fig. 4 shows the following elements:

### One or more ultrasound imaging systems 402

Ultrasound imaging systems proceed through their standard DICOM workflows, which includes study creation based on unique information about the patient, acquisition of series and images 404, and transfer of the primary images and secondary Structured Reports

### A database of medical images in the form of a DICOM storage server 406

The DICOM storage server may be any institutionally configured DICOM storage repository that supports Service Class Provider (SCP) and Service Class User (SCU).

### A patient monitoring device 200 (e.g. bedside monitor)

A device to visualize integrated data

### A patient monitoring model 408 (also referred to herein as a patient monitoring clinical decision support tool)

In the example in Fig. 4 the patient monitoring model is a Hemodynamic Stability Index (HSI) calculation model. As described above, the patient monitoring model performs a method that combines patient data together in order to determine a health parameter 410 (e.g. provide predictions and/or scores about the patient's health), and generates searchable strings 412 that can be used to perform queries into the DICOM space.

### A DICOM SCP and controller unit

### A unit to manage the flow of searchable strings and perform SCP queries

Matching image(s) from the query may be sent and displayed on the patient monitor 200 in step 414.
Thus, in this way, a patient monitoring system is able to filter and retrieve ultrasound images and measurements that are clinically relevant to a patient monitoring model's (e.g. algorithm's) output.

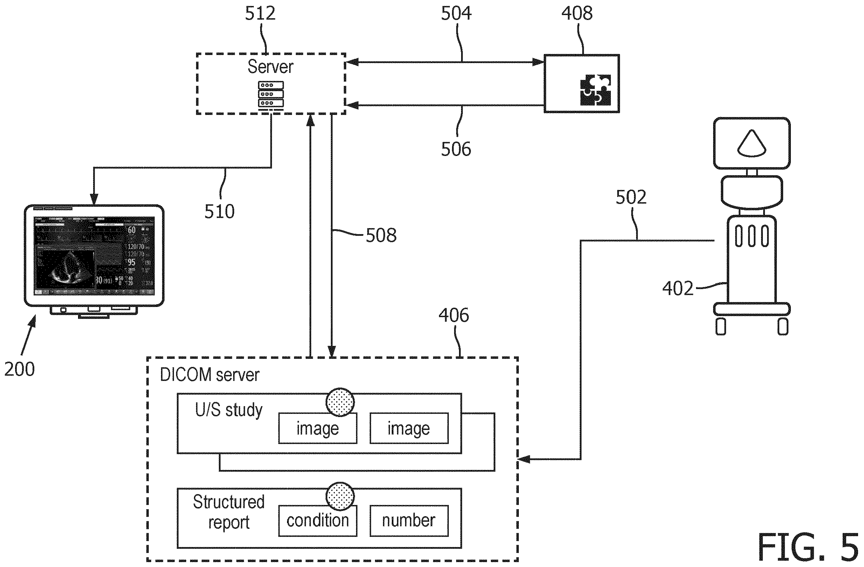

Turning now to Fig. 5, which shows an embodiment of the disclosure here. In this embodiment, the medical images are US images in the DICOM format and the patient monitoring model is for determining a HSI score. It will be appreciated that these are merely examples however and that the example in Fig. 5 applies equally to other image modalities and other patient monitoring models.

Step 502: The US acquisition system 402 stores images/measurements to the DICOM server 406. Ultrasound systems are used in accordance with standard DICOM workflow to generate studies, series, and images. Image processing processes and/or or expert reviewers produce measurements from these images to generate one or more observations that are encapsulated in Structured Reports. For a typical cardiac surgery patient in the ICU, three different ultrasound systems operated by different users produce six separate ultrasound studies with a total of 80 cine loops, containing 1600 image frames. Since this is a cardiac valve surgery patient, automated image analysis algorithms measure parameters such as Velocity Time Integral of the Aorta, and expert reviewers assess parameters such as mitral regurgitant volume (SR Code Meaning (0008,0104) aortic Regurgitation Volume (PISA)) with SR Finding Sites such as "left ventricular outflow tract" (CodeValue T-32650). These measurements are linked directly to SOP Instance UIDs for the secondary capture annotations produced by the algorithms. Numerous other measurements are made from the ultrasound data such as the Right Pulmonary Artery Diameter (Code Value 18021-6) on the Right Pulmonary Artery (Finding Site Code Value 44200).

Step 504: The patient monitoring server 512 and the HSI patient monitoring model 408 interact to generate predictions. The ultrasound system exports these studies and their associated reports for long term storage in a dedicated DICOM storage server, for example the institutional PACS. All six of the aforementioned studies could be exported and stored.

Step 506: The HSI patient monitoring model 408 exchanges data with the Patient Information Center 512 during the patient's stay and generates a prediction (e.g. according to step 302 above) that the patient's blood pressure will precipitously decrease within the next 15 minutes. The HSI patient monitoring model 408 recognizes that this event, in conjunction with the presence of a surgery and increasing heart rate likely relates to the patient's mitral valve function. The HSI patient monitoring model thus sends (in step 508) alongside its deterioration prediction, a list of DICOM query parameters. As described above, this list of parameters could be generated as part of the HSI patient monitoring model output, or performed separately by a predefined lookup table, or a user configurable lookup table (e.g. according to step 304 above).

Step 508: The patient monitoring controller uses the parameters to query the DICOM server, which returns a list of measurements and five image sequences (SOP Instances). In cases where measurements were made, includes image phase tags so specific parts of the cardiac cycle can be highlighted.

Step 510: The patient monitoring controller formats this list of image pointers to the bedside monitor 200, which renders the image according the data extracted from the DICOM server.

In the embodiment above, HSI was given as example for a predictive score derived from patient data. As noted above, this is merely an example and the system of Fig. 5 can work with another score, e.g. the hypotension prediction index (HPI) developed by Edwards. See paper by: Davies SJ, Vistisen ST, Jian Z, et al. entitled: "Ability of an arterial waveform analysis derived hypotension prediction index to predict future hypotensive events in surgical patients." Anesth Analg 2020;130:352-9. Also the papers by: Hatib F, Jian Z, Buddi S, et al. entitled: "Machine-learning algorithm to predict hypotension based on high-fidelity arterial pressure waveform analysis". Anesthesiology 2018; 129:663-74; and Wijnberge M, Geerts BF, Hol L, et al. "Effect of a machine learning derived early warning system for intraoperative hypotension vs standard care on depth and duration of intraoperative hypotension during elective noncardiac surgery". JAMA 2020;323:1052-60. Another example of a score that may be used is an acute heart failure prediction index, such as that described in the paper by Claire Zhao et al. entitled: "Predicting Disposition Decision, Mortality, and Readmission for Acute Heart Failure Patients in the Emergency Department Using Vital Sign, Laboratory, Echocardiographic, and Other Clinical Data" Circulation November 6, 2018 Vol 138, Issue Suppl_1.

Turning now to other embodiments, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method performed by a patient monitoring device, the method comprising:
using a patient monitoring model to determine a health parameter for a subject; and
using one or more outputs of the patient monitoring model to determine a search query with which to query a database of medical images in order to retrieve a matching medical image relevant to the determined health parameter.

2. A method as in claim 1 wherein the step of using one or more outputs of the patient monitoring model to determine a search query, is performed responsive:
to the determined health parameter triggering an alert on the patient monitoring device; and/or
to the determined health parameter being indicative of a deterioration of the patient.

3. A method as in claim 1 or 2 wherein medical images in the database of medical images are associated with accompanying structured reports comprising measurements and/or annotations of a feature in the associated medical image; and wherein the search query is formatted to search said measurements and/or annotations.

4. A method as in any one of claims 1 to 3 wherein at least one of the one or more outputs of the patient monitoring model is formatted as a string or image; and wherein the string or image is used in the search query.

5. A method as in any one of claims 1 to 3 wherein at least one of the one or more outputs of the patient monitoring model is formatted in a manner for use directly as a Digital Imaging and Communications in Medicine, DICOM, search query.

6. A method as in any one of the preceding claims wherein the step of using one or more outputs of the patient monitoring model to determine the search query comprises:
converting the one or more outputs of the patient monitoring model into the search query using: a look-up table, a machine learning model or a mapping procedure.

7. A method as in any one of the preceding claims wherein the determined health parameter is a score derived from patient data available to the patient monitoring device.

8. A method as in any one of the preceding claims wherein the score is:
a Hemodynamic Stability Index score;
a Hypotensive Predictive Index score;
an acute heart failure prediction index; or
a score linked to a patient monitoring alarm or patient monitoring trend.

9. A method as in any one of the preceding claims wherein the medical images contain meta information.

10. A method as in any one of the preceding claims wherein the medical images are in the DICOM image format.

11. A method as in any one of the preceding claims further comprising:
obtaining the matching medical image from the database of medical images; and
sending an instruction to a display to make the matching medical image available for rendering on the display.

12. An apparatus comprising:
a memory comprising instruction data representing a set of instructions; and
a processor configured to communicate with the memory and to execute the set of instructions, wherein the set of instructions, when executed by the processor, cause the processor to:
use a patient monitoring model to determine a health parameter for a subject; and
use one or more outputs of the patient monitoring model to determine a search query with which to query a database of medical images in order to retrieve a matching medical image relevant to the determined health parameter.

13. A patient monitoring device comprising the apparatus of claim 12, and further comprising:
a display; and wherein the processor is further caused to:
obtain the matching medical image from the database of medical images; and
send an instruction to the display to cause the display to make the matching medical image available for rendering on the display.

14. A patient monitoring device as in claim 13 wherein the processor is further caused to perform the method of any one of claims 2 to 10.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 11.
